# EUROPÄISCHE PATENTSCHRIFT

(11) **EP 3 255 039 B1**
(45) Veröffentlichungstag und Bekanntmachung des Hinweises auf die Patenterteilung: **12.12.2018**
(21) Anmeldenummer: 16173860.4
(22) Anmeldetag: 10.06.2016
(51) Int. Cl.: C07D 211/26, C07D 211/34

(54) **VERFAHREN ZUR HERSTELLUNG VON 2-(2,2,6,6-TETRAMETHYLPIPERIDIN-4-YL)PROPAN-1,3-DIAMIN**
METHOD FOR THE PREPARATION OF 2- (2,2,6,6-TETRAMETHYLPIPERIDINE-4-YL) PROPANE-1,3-DIAMINE
PROCEDE DE FABRICATION DE 2-(2, 2, 6, 6 - TETRAMETHYLPIPERIDINE-4-YL) PROPANE-1,3-DIAMINE

(43) Veröffentlichungstag der Anmeldung: 13.12.2017
(73) Patentinhaber: Evonik Degussa GmbH, 45128 Essen (DE)
(72) Erfinder: RÜFER, Alexander Martin, 45657 Recklinghausen (DE); RITTSTEIGER, Anne, 59399 Olfen (DE); NITZ, Jörg-Joachim, 45257 Essen (DE); KOHLSTRUK, Stephan, 45966 Gladbeck (DE); ORTELT, Martina, 74223 Flein (DE); FUCHSMANN, Dirk, 45721 Haltern am See (DE); DEMMING, Michael, 48249 Dülmen (DE); STEMMER, Christine, 45772 Marl (DE); OTT, Denise, 45772 Marl (DE); STASCH, Anja, 45657 Recklinghausen (DE)

(56) Entgegenhaltungen:
- WO-A1-2014/118121
- DE-A1- 1 695 749

## Beschreibung

Gegenstand der Erfindung ist ein Verfahren zur Herstellung von 2-(2,2,6,6-tetramethylpiperidin-4-yl)propan-1,3-diamin, welches als Härter in Epoxidanwendungen eingesetzt werden kann.

2-(2,2,6,6-tetramethylpiperidin-4-yl)propan-1,3-diamin, nachfolgend TMP-PDA genannt hat die chemische Struktur wie in Formel 1 dargestellt.

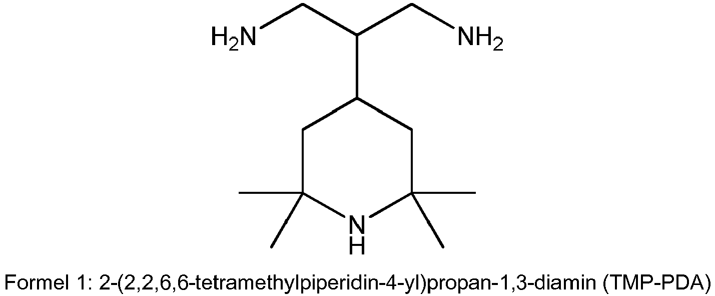

Es ist bekannt, dass Amine als Härter in Epoxidsystemen eingesetzt werden können. Epoxidharze sind Prepolymere, welche pro Molekül zwei oder mehr Epoxidgruppen enthalten. Die Reaktion dieser Harze mit einer Reihe von Härtungsmitteln führt zu vernetzten Polymeren. Ein Überblick über mögliche Harze und Härter sowie deren Verwendung und Eigenschaften wird in H. Schumann, "Handbuch Betonschutz durch Beschichtung", Expert Verlag 1992, Seiten 396-428 gegeben.

Aufgabe war es, ein Verfahren zur Herstellung von 2-(2,2,6,6-tetramethylpiperidin-4-yl)propan-1,3-diamin zu finden.

Aufgabe war es auch, ein neues Amin zu finden, welches für die Härtung von Epoxidsystemen geeignet ist.

Gegenstand der Erfindung ist ein Verfahren zur Herstellung von 2-(2,2,6,6-tetramethylpiperidin-4-yl)propan-1,3-diamin, durch
A) Umsetzung von Triacetonamin (TAA) und Malononitril zum Zwischenprodukt 2-(2,2,6,6-tetramethylpiperidin-4-yliden)malononitril, und
B) Hydrierung von 2-(2,2,6,6-tetramethylpiperidin-4-yliden)malononitril in Gegenwart mindestens eines Katalysators.

Die Herstellung der erfindungsgemäßen Verbindung 2-(2,2,6,6-tetramethylpiperidin-4-yl)propan-1,3-diamin erfolgt im Schritt A) über eine Knoevenagelkondensation zwischen Triacetonamin (TAA) und Malononitril. Die Reaktion kann im Lösungsmittel oder in einem lösungsmittelfreien Reaktionssystem unter milden Reaktionsbedingungen durchgeführt werden, bevorzugt bei 20-40°C und Normaldruck. Als Katalysator wird bevorzugt Zirkonylchlorid oder Piperidin eingesetzt. Nach vollständiger Umsetzung der Reaktanden kann das Zwischenprodukt 2-(2,2,6,6-tetramethylpiperidin-4-yliden)malononitril (vgl. Struktur Formel 2 in Beispiel 1) durch Abkühlen der Reaktionslösung als Feststoff ausgefällt werden. Eine weitere Aufreinigung kann z.B. durch Destillation erfolgen.

Die Herstellung von TMP-PDA aus 2-(2,2,6,6-tetramethylpiperidin-4-yliden)malononitril erfolgt im Schritt B) über Hydrierung, die in einer oder mehreren Stufen durchgeführt werden kann. Bei der Verwendung von mehreren Hydrierungsreaktionen können die einzelnen Stufen in einem Reaktor mit unterschiedlichen Katalysatorzonen oder in mehreren separaten oder in Reihe geschalteten Reaktoren durchgeführt werden. Bevorzugt erfolgt die Hydrierung in Festbettreaktoren. Geeignete Reaktortypen sind z.B. Schachtöfen, Hordenreaktoren oder Rohrbündelreaktoren. Es ist auch möglich, für die Hydrierung mehrere Festbettreaktoren hintereinander zu schalten, wobei jeder der Reaktoren wahlweise in Rieselbett- und Sumpffahrweise betrieben wird.

Als Katalysatoren können prinzipiell alle Katalysatoren eingesetzt werden, die die Hydrierung von Nitrilgruppen mit Wasserstoff katalysieren. Besonders geeignet sind Nickel-, Kupfer-, Eisen-, Palladium-, Rhodium-, Ruthenium- und Cobaltkatalysatoren, ganz besonders Palladium-Ruthenium- und Cobaltkatalysatoren. Zur Erhöhung der Aktivität, Selektivität und/oder Standzeit können die Katalysatoren zusätzliche Dotiermetalle oder andere Modifizierungsmittel enthalten. Typische Dotiermetalle sind z.B. Mo, Fe, Ag, Cr, Ni, V, Ga, In, Bi, Ti, Zr und Mn sowie die seltenen Erden. Typische Modifizierungsmittel sind z. B. solche, mit denen die Säure-Base-Eigenschaften der Katalysatoren beeinflusst werden können, bevorzugt Alkali- und Erdalkalimetalle bzw. deren Verbindungen, bevorzugt Mg- und Ca-Verbindungen, sowie Phosphorsäure oder Schwefelsäure sowie deren Verbindungen.

Die Katalysatoren können in Form von Pulvern oder Formkörpern, wie z.B. Extrudaten oder gepressten Pulvern, eingesetzt werden. Es können Vollkontakte, Raney-Typ-Katalysatoren oder Trägerkatalysatoren zur Anwendung kommen. Bevorzugt sind Raney-Typ- und Trägerkatalysatoren. Geeignete Trägermaterialien sind z.B. Siliciumdioxid, Aluminiumoxid, Alumosilikate, Titandioxid, Zirkoniumdioxid, Kieselgur, Aluminium-Silicium-Mischoxide, Magnesiumoxid und Aktivkohle. Das Aktivmetall kann in einer dem Fachmann bekannten Weise auf das Trägermaterial aufgebracht werden, wie z.B. durch Imprägnierung, Aufsprühen oder Fällung. Je nach Art der Katalysatorherstellung sind weitere, dem Fachmann bekannte, Präparationsschritte notwendig, wie z.B. Trocknung, Calcinierung, Formgebung und Aktivierung. Zur Formgebung können optional weitere Hilfsstoffe wie z.B. Graphit oder Magnesiumstearat zugesetzt werden. Das erforderliche Volumen der einzusetzenden Hydrierkatalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung zu gewährleisten.

Für die Herstellung des erfindungsgemäßen Diamins TMP-PDA werden bevorzugt Hydrierkatalysatoren auf der Basis von Palladium und/oder Cobalt eingesetzt. Diese Katalysatoren weisen eine besonders gute Aktivität zur Erreichung einer hohen Ausbeute auf. Die Katalysatoren können in Form von Pulvern oder Festbettkatalysatoren eingesetzt werden. Die Hydrierung kann in Batch-Fahrweise oder in kontinuierlich betriebenen Anlagen erfolgen. Die Reaktionsbedingungen für die Hydrierung liegen zwischen 20-120°C und 20-300 bar.

Die Hydrierung kann in ein oder mehreren Stufen durchgeführt werden. Bevorzugt wird die Hydrierung zweistufig durchgeführt. Hierbei werden in der ersten Stufe Reaktionsbedingungen von 20-120°C und 20-300 bar, bevorzugt 40-100°C und 25-150 bar und besonders bevorzugt 60-90°C und 40-80 bar gewählt. In der zweiten Stufe der Hydrierung werden Reaktionsbedingungen von 20-120°C und 20-300 bar, bevorzugt 50-115°C und 50-200 bar und besonders bevorzugt 80-110°C und 80-140 bar gewählt.
In der ersten Stufe der Hydrierung wird bevorzugt ein Palladium-Katalysator eingesetzt.

In der zweiten Stufe der Hydrierung wird bevorzugt ein Raney-Typ-Katalysator eingesetzt.
Der Katalysator weist besonders bevorzugt nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

und
mit Partikelgrößen des Katalysators, also der Granulat-Teilchen mit einer statistischen Verteilung zwischen 3 bis 7 Millimetern (mm), wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

Das die Hydrierung verlassende Reaktionsgemisch wird mit den üblichen Methoden weiter aufgereinigt, um ein TMP-PDA mit der gewünschten Qualität zu erhalten. Dabei können alle gängigen Trennmethoden wie z. B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich, absatzweise, ein- oder mehrstufig, im Vakuum oder unter Druck durchgeführt werden. Die Aufreinigung von TMP-PDA wird bevorzugt z.B. durch Destillation durchgeführt.

Bevorzugt wird die Aufreinigung durch Destillation unter Druck und/oder im Vakuum in mehreren Schritten erreicht. Hierfür lassen sich beliebige Destillationskolonnen mit und ohne Einbauten wie z. B. Dephlegmatoren, Trennwänden, ungeordnete Einbauten oder Schüttungen, geordnete Einbauten oder Packungen, Böden mit oder ohne Zwangsführung verwenden.

### Anwendung als Epoxidhärter:

Gegenstand der Erfindung ist auch die Verwendung von 2-(2,2,6,6-tetramethylpiperidin-4-yl)propane-1,3-diamine (TMP-PDA) als Härter in Epoxidharzzusammmensetzungen.

Als Epoxidharzkomponente kommen prinzipiell alle Epoxidharze in Frage, die mit Aminen gehärtet werden können. Zu den Epoxidharzen zählen z.B. Polyepoxide auf Basis von Bisphenol A-diglycidylether, Bisphenol F-diglycidylether oder cycloaliphatische Typen. Vorzugsweise werden jedoch Epoxidharze auf Basis Bisphenol A und gegebenenfalls die auf Basis Bisphenol F - ggf. auch in Mischung - eingesetzt. Harze und Härter werden dabei bevorzugt in äquivalenten Mengen eingesetzt. Es sind jedoch auch Abweichungen vom stöchiometrischen Verhältnis möglich.

### Beispiele

### Beispiel 1: Herstellung von 2-(2,2,6,6-tetramethylpiperidin-4-yl)propan-1,3-diamin, TMP-PDA

Schritt A): Synthese von 2-(2,2,6,6-tetramethylpiperidin-4-yliden)malononitril. 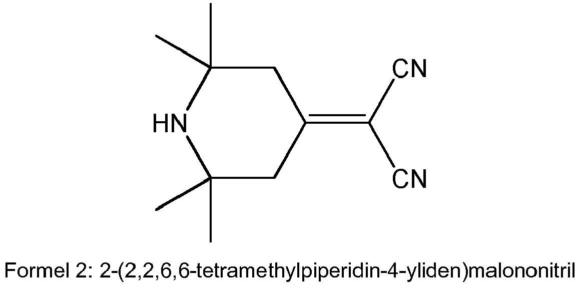
- In einen 1 L-Dreihalskolben mit zwei Tropftrichtern wurde 384,5 g Triacetonamin (TAA) in 350 g Dioxan vorgelegt. Der Reaktorinhalt wurde dabei auf Raumtemperatur gehalten.
- 148,5 g Malononitril wurden mit 150 g Dioxan verdünnt und in einem Tropftrichter vorgelegt.
- 1,5 g Zirkonylchlorid (Katalysator) wurden in 30 g entionisiertem Wasser gelöst und in den zweiten Tropftrichter gefüllt.
- Anschließend wurde der Inhalt beider Tropftrichter simultan tropfenweise in den Reaktor gegeben und der Reaktor im Anschluss für eine Stunde bei Raumtemperatur (zwischen 20-30°C) gerührt.
- Die entstandene Reaktionsmischung wurde mit einem Eisbad gekühlt und das dabei ausgefallende Produkt 2-(2,2,6,6-tetramethylpiperidin-4-yliden)malononitril abfiltriert.
- Die weitere Aufreinigung erfolgte über eine Umkristallisation in einer Lösungsmittelmischung aus 50 Gew.-% kaltem Ethanol und 50 Gew.-% kaltem entionisiertem Wasser und einer anschließenden Filtration und Trocknung im Vakuumtrockenschrank (45°C, 10 mbar, 3 h).
- Die Produktzusammensetzung wurde mittels Gaschromatographie ermittelt.

Die Ausbeute betrug 90% an 2-(2,2,6,6-tetramethylpiperidin-4-yliden)malononitril.

Schritt B1): Teilhydrierung von 2-(2,2,6,6-tetramethylpiperidin-4-yliden)malononitril zur Herstellung von TMP-PDA, 1. Hydrierstufe.
- In einen 2 L-Druckautoklaven mit Katalysatorkorb wurden 150 ml des Festbettkatalysators Pd/Aluminiumoxid (1 Gew.-% Pd) eingebaut.
- Für die Reaktion wurde 1 L Lösung mit 10 Gew.-% 2-(2,2,6,6-tetramethylpiperidin-4-yliden)malononitril (Produkt aus Schritt A) in Tetrahydrofuran vorgelegt.
- Die Reaktion erfolgte bei 75°C, 50 bar Wasserstoff für 5 h.
- Die komplette Produktlösung wurde aus dem Reaktor abgelassen.
- Die Zusammensetzung der Produktlösung wurde mittels Gaschromatographie bestimmt.

### Schritt B2, 2. Hydrierstufe): Vollhydrierung von Produktlösung aus Schritt B1,

In einen 2 L-Druckautoklaven mit Katalysatorkorb wurden 150 ml aktivierte Cobalt-Legierungsgranulate nach Raney als Festbett eingebaut. Der Katalysator wies dabei die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 75,9 Gew.-% |
| Aluminium: | 20,0 Gew.-% |
| Chrom: | 1,5 Gew.-% |
| Nickel: | 2,6 Gew.-% |

Es wurde eine Siebfraktion des Katalysators mit einer statistischen Verteilung zwischen 2,0 und 5,0 Millimetern (mm) eingesetzt, wobei bis zu 10% der Partikel oberhalb der genannten Obergrenze und bis zu 10% der Partikel unterhalb der genannten Untergrenze liegen können.
- Für die Reaktion wurde 1 L Reaktionslösung (teilhydriertes Produkt aus Schritt B1 in THF) vorgelegt.
- Die Reaktion erfolgte bei 100°C, 100 bar Wasserstoff für 5 h.
- Die Zusammensetzung der Produktlösung wurde mittels Gaschromatographie ermittelt.
- Für den Einsatz von TMP-PDA als Härter in Epoxidharzsystemen wurde das erhaltene Produkt mittels Destillation gereinigt.

Die Ausbeute der zweistufigen Hydrierung betrug 85 Gew.-% TMP-PDA bezogen auf die eingesetzte Masse 2-(2,2,6,6-tetramethylpiperidin-4-yliden)malononitril aus Schritt A.

### Beispiel 2: TMP-PDA als Härter in Epoxidharzsystemen

Als Epoxidharz wurde das Standardharz Epikote 828 der Firma Hexion mit EpoxidÄquivalentgewicht 188 g/val verwendet. Dieses wurde in stöchiometrischer Übereinstimmung der H-Äquivalente mit der Härterkomponente TMP-PDA vermengt (vgl. Tabelle 1) und die Glasübergangstemperatur (Tg) nach einer Verweilzeit von einer Stunde bei definierter Härtungstemperatur bestimmt (Tabelle 2). Die jeweiligen Reaktionsumsätze wurden über die aufgenommene Wärmetönung der Härtungsreaktion in Relation zur Maximalwärmetönung bestimmt (Tabelle 3).

**Tabelle 1: Verhältnis von Harz zu Härter**

| | |
|---|---|
| Härterkomponente TMP-PDA (g) | 100 |
| Menge Epoxidharz (g) pro 100 g Härter | 441 |

**Tabelle 2: Glasübergangstemperaturen (Tg) nach 1 h Härtung bei verschiedenen Temperaturen**

| | |
|---|---|
| Tgmax. (DSC) | 175°C |
| Tg nach 1 h 50°C | 37°C |
| Tg nach 1 h 70°C | 76°C |
| Tg nach 1 h 90°C | 102°C |
| Tg nach 1 h 110°C | 128°C |
| Tg nach 1 h 130°C | 150°C |
| Tg nach 1 h 150°C | 162°C |

**Tabelle 3: Umsätze**

| | |
|---|---|
| Umsatz nach 1 h 50°C | 62 % |
| Umsatz nach 1 h 70°C | 80 % |
| Umsatz nach 1 h 90°C | 90 % |
| Umsatz nach 1 h 110°C | 95% |
| Umsatz nach 1 h 130°C | 97% |
| Umsatznach 1 h 150°C | 100% |

Wie der Fachmann anhand Tabelle 1, Tabelle 2 und Tabelle 3 leicht erkennen kann, eignet sich TMP-PDA als Härterkomponente in Epoxidharzsystemen.

## Patentansprüche

1. Verfahren zur Herstellung von 2-(2,2,6,6-tetramethylpiperidin-4-yl)propane-1,3-diamine, durch
A) Umsetzung von Triacetonamin (TAA) und Malononitril zum Zwischenprodukt 2-(2,2,6,6-tetramethylpiperidin-4-yliden)malononitril,
und
B) Hydrierung von 2-(2,2,6,6-tetramethylpiperidin-4-yliden)malononitril in Gegenwart mindestens eines Katalysators.

2. Verfahren nach Anspruch 1, wobei die Hydrierung im Schritt B) bei 20-120°C und bei 20-300 bar durchgeführt wird.

3. Verfahren nach Anspruch 1, wobei die Hydrierung im Schritt B) in zwei Stufen bei 20-120°C und bei 20-300 bar durchgeführt wird.

4. Verfahren nach Anspruch 3, wobei die Hydrierung im Schritt B) in zwei Stufen bei 20-120°C und bei 20-300 bar durchgeführt wird.

5. Verfahren nach Anspruch 3-4, wobei die Hydrierung in der ersten Stufe bei 40-100°C und 25-150 bar, und in der zweiten Stufe bei 50-115°C und 50-200 bar durchgeführt wird.

6. Verfahren nach Anspruch 3-4, wobei die Hydrierung in der ersten Stufe bei 60-90°C und 40-80 bar, und in der zweiten Stufe bei 80-110°C und 80-140 bar durchgeführt wird.

7. Verfahren nach mindestens einem der vorherigen Ansprüche, **dadurch gekennzeichnet, dass** Katalysatoren ausgewählt aus Nickel-, Kupfer-, Eisen-, Palladium-, Rhodium-, Ruthenium- und Cobaltkatalysatoren, eingesetzt werden.

8. Verfahren nach mindestens einem der vorherigen Ansprüche, **dadurch gekennzeichnet, dass** als Katalysatoren Palladium und/oder Cobaltkatalysatoren eingesetzt werden.

9. Verfahren nach mindestens einem der vorherigen Ansprüche, **dadurch gekennzeichnet, dass** als Katalysatoren Raney-Typ-Katalysatoren oder Trägerkatalysatoren eingesetzt werden.

10. Verfahren nach mindestens einem der vorherigen Ansprüche, **dadurch gekennzeichnet, dass** als Katalysator ein Katalysator aus aktivierten Cobalt-Legierungsgranulaten nach Raney eingesetzt wird, wobei der Katalysator nach der Aktivierung in seiner Gesamtheit die folgende Zusammensetzung in Gewichtsprozent (Gew.-%) aufweist, wobei sich die Anteile zu 100 Gew.-% addieren, bezogen auf die enthaltenden Metalle:
| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |
und
mit Partikelgrößen des Katalysators, also die Granulat-Teilchen mit einer statistischen Verteilung zwischen 3 bis 7 Millimeter (mm), wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

11. Verfahren nach mindestens einem der vorherigen Ansprüche, **dadurch gekennzeichnet, dass** die Hydrierung in Festbettreaktoren, bevorzugt in Schachtöfen, Hordenreaktoren oder Rohrbündelreaktoren, durchgeführt wird.

12. Verwendung von 2-(2,2,6,6-tetramethylpiperidin-4-yl)propan-1,3-diamin als Härter in Epoxidharzzusammmensetzungen.

## Claims

1. Process for producing 2-(2,2,6,6-tetramethylpiperidin-4-yl)propane-1,3-diamine by
A) reacting triacetonamine (TAA) and malononitrile to afford the intermediate 2-(2,2,6,6-tetramethylpiperidin-4-ylidene)malononitrile,
and
B) hydrogenating 2-(2,2,6,6-tetramethylpiperidin-4-ylidene)malononitrile in the presence of at least one catalyst.

2. Process according to Claim 1, wherein the hydrogenation in step B) is performed at 20-120°C and at 20-300 bar.

3. Process according to Claim 1, wherein the hydrogenation in step B) is performed in two stages at 20-120°C and at 20-300 bar.

4. Process according to Claim 3, wherein the hydrogenation in step B) is performed in two stages at 20-120°C and at 20-300 bar.

5. Process according to Claims 3-4, wherein the hydrogenation is performed at 40-100°C and 25-150 bar in the first stage and at 50-115°C and 50-200 bar in the second stage.

6. Process according to Claims 3-4, wherein the hydrogenation is performed at 60-90°C and 40-80 bar in the first stage and at 80-110°C and 80-140 bar in the second stage.

7. Process according to at least one of the preceding claims, **characterized in that** catalysts selected from nickel, copper, iron, palladium, rhodium, ruthenium and cobalt catalysts are employed.

8. Process according to at least one of the preceding claims, **characterized in that** the catalysts employed are palladium and/or cobalt catalysts.

9. Process according to at least one of the preceding claims, **characterized in that** the catalysts employed are Raney-type catalysts or supported catalysts.

10. Process according to at least one of the preceding claims, **characterized in that** the catalyst employed is a catalyst composed of activated Raney cobalt alloy pellets, wherein after activation the catalyst in its entirety has the following composition in weight per cent (wt%), the proportions summing to 100 wt% based on the metals present:
| | |
|---|---|
| cobalt: | 57 to 84 wt% |
| aluminium: | 10 to 40 wt% |
| chromium: | 1 to 2 wt% |
| nickel: | 2 to 4 wt% |
and
with particle sizes of the catalyst, i.e. the pellet particles, having a statistical distribution between 3 to 7 millimetres (mm), wherein up to 10 per cent of the particles may also be outside the stated range of the stated lower limit or upper limit but also in each case up to 10 per cent may be outside the stated range of the stated lower limit and upper limit.

11. Process according to at least one of the preceding claims, **characterized in that** the hydrogenation is performed in fixed-bed reactors, preferably in shaft furnaces, tray reactors or shell and tube reactors.

12. Use of 2-(2,2,6,6-tetramethylpiperidin-4-yl)propane-1,3-diamine as a hardener in epoxy resin compositions.

## Revendications

1. Procédé pour la production de 2-(2,2,6,6-tétraméthylpipéridin-4-yl)propane-1,3-diamine par
A) la réaction de triacétonamine (TAA) et de malononitrile pour fournir l'intermédiaire 2-(2,2,6,6-tétraméthylpipéridin-4-ylidène)malononitrile
et
B) l'hydrogénation de 2-(2,2,6,6-tétraméthylpipéridin-4-ylidène)malononitrile en présence d'au moins un catalyseur.

2. Procédé selon la revendication 1, dans lequel l'hydrogénation dans l'étape B) est effectuée à 20-120 °C et à 20-300 bar.

3. Procédé selon la revendication 1, dans lequel l'hydrogénation dans l'étape B) est effectuée en deux étapes à 20-120 °C et à 20-300 bar.

4. Procédé selon la revendication 3, dans lequel l'hydrogénation dans l'étape B) est effectuée en deux étapes à 20-120 °C et à 20-300 bar.

5. Procédé selon les revendications 3-4, dans lequel l'hydrogénation est effectuée à 40-100 °C et 25-150 bar dans la première étape et à 50-115 °C et 50-200 bar dans la seconde étape.

6. Procédé selon les revendications 3-4, dans lequel l'hydrogénation est effectuée à 60-90 °C et 40-80 bar dans la première étape et à 80-110 °C et 80-140 bar dans la seconde étape.

7. Procédé selon au moins l'une des revendications précédentes, **caractérisé en ce que** des catalyseurs choisis parmi les catalyseurs au nickel, au cuivre, au fer, au palladium, au rhodium, au ruthénium et au cobalt sont employés.

8. Procédé selon au moins l'une des revendications précédentes, **caractérisé en ce que** les catalyseurs employés sont des catalyseurs au palladium et/ou au cobalt.

9. Procédé selon au moins l'une des revendications précédentes, **caractérisé en ce que** les catalyseurs employés sont des catalyseurs de type Raney ou des catalyseurs supportés.

10. Procédé selon au moins l'une des revendications précédentes, **caractérisé en ce que** le catalyseur employé est un catalyseur composé de granulés d'alliage de cobalt de Raney activé, le catalyseur dans sa globalité après activation ayant la composition suivante en pourcentage en poids (% en poids), les proportions totalisant 100 % en poids par rapport aux métaux présents :
| | |
|---|---|
| cobalt : | 57 à 84 % en poids |
| aluminium : | 10 à 40 % en poids |
| chrome : | 1 à 2 % en poids |
| nickel : | 2 à 4 % en poids |
et
les tailles des particules du catalyseur, c'est-à-dire des granulés de catalyseur, ayant une distribution statistique comprise entre 3 et 7 millimètres (mm), jusqu'à 10 pour cent des particules pouvant également être à l'extérieur de la plage indiquée du côté de la limite inférieure ou de la limite supérieure indiquées mais également dans chaque cas jusqu'à 10 pour cent pouvant être à l'extérieur de la plage indiquée du côté de la limite inférieure et de la limite supérieure indiquées.

11. Procédé selon au moins l'une des revendications précédentes, **caractérisé en ce que** l'hydrogénation est effectuée dans des réacteurs à lit fixe, de préférence dans des fours à cuve, des réacteurs à plateaux ou des réacteurs tubulaires à calandre.

12. Utilisation de 2-(2,2,6,6-tétraméthylpipéridin-4-yl)propane-1,3-diamine en tant que durcisseur dans des compositions de résine époxy.
